# EUROPEAN PATENT SPECIFICATION

(11) **EP 1 082 908 B1**
(45) Date of publication and mention of the grant of the patent: **26.08.2009**
(21) Application number: 00911297.0
(22) Date of filing: 23.03.2000
(51) Int. Cl.: A23K 1/16, A23K 1/18, A61K 31/739, A61K 31/00, A61K 38/16

(54) **ADDITIVES FOR CRUSTACEAN OR FISH FEEDS AND FEEDS**
ZUSAETZE FUER KRUSTENTIER- BZW. FISCHFUTTERMITTEL SOWIE FUTTERMITTEL
ADDITIFS POUR ALIMENTS DE CRUSTACEES OU DE POISSONS ET ALIMENTS

(30) Priority: 26.03.1999 JP 8439999
(43) Date of publication of application: 14.03.2001
(73) Proprietor: Soma, Genichiro, Setagaya-ku Tokyo 158-0084 (JP); Takahashi, Yukinori, Shimonoseki-shi Yamaguchi 751-0856 (JP)
(72) Inventor: SOMA, Genichiro, Setagaya-ku, Tokyo 158-0084 (JP); TAKAHASHI, Yukinori, Shimonoseki-shi, Yamaguchi 751-0856 (JP); MIZUNO, Denichi, Kamakura-shi, Kanagawa 247-0072 (JP)
(74) Representative: Barz, Peter
(86) International application number: PCT/JP2000/001764
(87) International publication number: WO 2000/057719

(56) References cited:
- EP-A2- 0 472 467
- EP-A2- 0 592 220
- WO-A1-96/23002
- JP-A- 06 141 849
- JP-A- 08 280 332
- JP-A- 10 279 486
- VASSILIS BAKOPOULOS ET AL.: "Qualitative differences in the immune response of rabbit, mouse and sea bass, Dicentrarchus labrax, L. to Photobacterium damsela subsp. piscicida, the causative agent of fish Pasteurellosis" FISH AND SHELLFISH IMMUNOLOGY, vol. 7, no. 3, 1997, pages 161-174, XP002292157 GBACADEMIC PRESS, LONDON,
- YUKINORI TAKAHASHI YOUSHOKU vol. 34, no. 10, 1997, pages 117 - 121, XP002935493
- FULVIO SALATI ET AL.: 'Effect of Edwardsiella tarda Lipopolysaccharide Immunization on Phagocytosis in the Eel' NIPPON SUISAN GAKKAISHI vol. 53, no. 2, 1987, pages 201 - 204, XP002928719
- MARILYN J. ODEAN ET AL.: 'Involvement of Gamma Interferon in Antibody Enhancement by Adjuvants' INFECTION AND IMMUNITY vol. 58, no. 2, 1990, pages 427 - 432, XP002928720
- L.W. CLEM ET AL.: 'MONOCYTES AS ACCESSORY CELLS IN FISH IMMUNE RESPONSES' DEVELOPMENT AND COMPARATIVE IMMUNOLOGY vol. 9, 1985, pages 803 - 809, XP002928721

## Description

### TECHNICAL FIELD

The present invention relates to a feedstuff additive for crustaceans or fishes, and a feed containing the feedstuff additive, and more particularly to a feedstuff additive which shows significant effects of activating immunity and preventing infection and to a feed containing the same in a suitable proportion.

### BACKGROUND ART

Recent years have seen development of aquiculture of crustaceans and fishes. Attendant on the development is a great economical damage in the culture industry due to outbreaks of bacterial or viral diseases of crustaceans and fishes. Diseases of crustaceans and fishes often occurring include acute viremia of kuruma prawns (Penaeus japonicus), vibriosis thereof, pseudotuberculosis of yellowtails, enterococcus diseases thereof, cold-water disease of sweet fishes (ayu), Pseudomonas diseases thereof, iridovirus diseases of red sea breams, Seriola dumerili, yellowtails or the like which have economically damaged the culture industry. Of these diseases, bacterial diseases have been treated with antibiotics or synthetic antibacterial agents as a curative agent. However, with the advent of antibiotic-resistant bacteria, satisfactory curative effects have not been achieved. Further, a problem of public health hazards has been raised because of the medicinal agent remaining in crustaceans and fishes. Consequently, there is a strong demand for preventive measures not depending on chemotherapy. On the other hand, vaccines and curative agents have not been developed against viral diseases of crustaceans and fishes and viral diseases still often occur.

The use of polysaccharides is already known to immunopotentiate crustaceans and fishes and to prevent infectious diseases thereof. These polysaccharides include, for example, peptidoglycan derived from Bifidobacterium thermophilum (Patent No.2547371), cell wall-forming component of gram-positive bacteria like bacteria of genus Bacillus (JP-B-3-173826) and β-1,3-glucan derived from Schizophyllum commune (JP-B-6-65649). It was already reported that high molecular weight lipopolysaccharides activate the immune function of fishes and animals (Salati, F. and R. Kusuda, Society Journal, Japanese Society of Science of Fisheries, vol.53, pp.201 to 204, 1987 and Odean, M.J. et al., Infection and Immunity, vol.58, pp.427 to 432, 1990).

On the other hand, the low molecular weight lipopolysaccharide of the present invention (hereinafter referred to as "low molecular weight LPS") is different in basic structure and components from the peptidoglycan derived from gram-positive bacteria, cell wall-forming component and β -1,3-glucan derived from a mushroom. The low molecular weight LPS of the invention comprises three components, i.e. a specific lipid A, an oligosaccharide with covalent bond therewith called R core and O specific polysaccharide. The low molecular weight LPS of the invention is known as an immunopotentiator for animals because of its ability to increase the tumor necrosis factor (TNF)-producing effect, but is not known at all to have an activity of preventing infectious diseases of crustaceans and fishes. The high molecular weight lipopolysaccharides (LPSs) used in the researches heretofore reported are those with a markedly high molecular weight as high as 1 million to 10 millions and are of high toxicity. Consequently, when applied to crustaceans and fishes for a long period, such high molecular weight LPS is unable to activate the immune function all the time. The above-mentioned known substances have a high molecular weight and need to be orally administered in a large quantity because of their poor absorption through the intestinal tract. Consequently, a long-period intake of them frequently results in impairment of immune function.

As described above, a variety of infectious diseases often occur in crustaceans and fishes. Some of these diseases are lethal and may result in great economic damage. The background to be noted is that the immune function of crustaceans and fishes is deteriorated because they are bred in an overcrowded area under a limited environment. Various substances were used to reactivate their impaired immune system. On the other hand, crustaceans have no ability to produce an antibody nor lymphocyte, neutrophile or basophile as found in a vertebrate. Fishes have a limited ability to produce an antibody and its production of antibody is greatly affected by the temperature of water because they are cold-blooded animals so that such immune system is not sufficiently functioned. In other words, substantial difference exists in defensive mechanism between these oceanic organisms and mammals (Fish Pathology, 30(2), 141-150, June in 1995). Consequently some of the substances are not usable in-situ in breeding oceanic organisms because of high toxicity like conventional LPSs, and most of them are impaired in the immune system by intake of the LPSs for a prolonged period.

WO-A-9623002 discloses low molecular weight lipopolysaccharides as immuno-modulators.

An object of the present invention is to provide a safe feedstuff additive for culture or breeding of crustaceans and fishes, the feedstuff additive being capable of preventing infectious diseases even in a small amount by properly activating their intrinsic immune function, and being free from problems of public health hazards such as the feedstuff additive remaining in crustaceans and fishes.

### DISCLOSURE OF THE INVENTION

The present invention provides a feedstuff additive for crustaceans and fishes, **characterized in that** it is prepared from gram-negative bacteria, that it has a molecular weight of 5000 ± 2000 as measured by SDS-PAGE method using a protein marker, that it is substantially free of high molecular weight lipopolysaccharide, and that it contains a low molecular weight lipopolysaccharide as an effective component and that it is capable of activating immunity or preventing infection in crustaceans or fishes; and a feed for crustaceans or fishes which feed is **characterized in that** it contains the feedstuff additive.

The present invention also provides a feedstuff additive for crustaceans or fishes comprising the low molecular weight lipopolysaccharide and a carrier acceptable for crustaceans and fishes.

The present invention also provides use of the low molecular weight lipopolysaccharide for the preparation of a feedstuff additive for crustaceans or fishes.

The present invention also provides a method of activating immunity or preventing infection in crustaceans and fishes comprising administering an effective amount of the low molecular weight lipopolysaccharide to crustaceans or fishes.

The present invention also provides an agent for preventing the perish of crustaceans or fishes comprising the low molecular weight lipopolysaccharide as an effective component.

The present invention also provides an agent for preventing the perish of crustaceans or fishes comprising the low molecular weight lipopolysaccharide and a carrier acceptable for crustaceans and fishes.

The present invention also provides use of the low molecular weight lipopolysaccharide of for the preparation of an agent for preventing the perish of crustaceans or fishes.

The present invention also provides an agent for preventing the perish of crustaceans or fishes comprising administering an effective amount of the low molecular weight lipopolysacchride to crustaceans or fishes.

The present invention also provides a feedstuff additive, wherein the gram-negative bacteria are those pertaining to genus Pantoea.

The present invention also provides a feedstuff additive, wherein the gram-negative bacteria are Pantoea agglomerans.

The present invention also provides a feed for crustaceans or fishes comprising the feedstuff additive.

The present invention also provides a feed for crustaceans or fishes comprising the agent for preventing the perish.

The present invention also provides a method of breeding crustaceans or fishes comprising administering the feed to crustaceans or fishes.

The feedstuff additive of the invention is prepared from gram-negative bacteria by purification, e.g. according to the method disclosed in JP-A-8-198902. The present inventors prepared a feed containing a low molecular weight LPS having a molecular weight of 5000 ± 2000. When the feed was supplied to crustaceans and fishes, it was found that the feed prevented viral or bacterial infectious diseases and protected them against decease by activation of the intrinsic immune function. The present invention was accomplished based on this finding.

The low molecular weight LPS of the present invention is, as described above, a lipopolysaccharide having a molecular weight of 5000 ± 2000 which is prepared from gram-negative bacteria, e.g. according to the method disclosed in JP-A-8-198902. The LPS of this invention is **characterized in that** the LPS is pronouncedly safer for crustaceans or fishes and can produce a significantly higher effect of activating immunity and a higher effect of preventing infection and decease than conventional LPSs (with a molecular weight of 1 million to 10 millions).

In the present invention, the term "substantially free of high molecular weight lipopolysaccharide" means "not containing lipopolysaccharide having a molecular weight of at least 8,000".

The gram-negative bacteria for use in the invention include, for example, those pertaining to genera Pantoea, Salmonella, Aeromonas, Serratia and Enterobacter, and further include those described in JP-A-4-99481. Among useful gram-negative bacteria, those of Pantoea are preferred and those of Pantoea agglomerans are more preferred.

The low molecular weight LPS of the present invention can be prepared by a method comprising incubating gram-negative bacteria or the like in the conventional manner, collecting the cultured bacteria from the culture medium, extracting the collected bacteria by conventional methods, such as hot phenol method (edited by O. Westphal, Methods in Carbohydrate Chemistry, vol. 5, p.83, Academic Press, 1965) and purifying the extract with an anion exchange resin. More specifically, the method comprises suspending bacteria in distilled water, adding the suspension to a mixture of distilled water and an equal volume of hot phenol, stirring the mixture, centrifuging the mixture to recover the aqueous layer, dialyzing the aqueous layer to remove the phenol, concentrating the aqueous layer by ultrafiltration to obtain crude LPS fractions, purifying the fractions by conventional anion exchange chromatography (e.g. using mono Q-Sepharose or Q-Sepharose) and desalting the same in the conventional manner.

The purified LPS thus obtained is substantially identical with the LPSs having a molecular weight of about 5,000 to about 6,000 as disclosed in JP-A-4-187640, JP-A-4-49240, JP-A-4-99481 and JP-A-5-155778. The purified LPS is subjected to gel filtration in the presence of a surface-active agent such as sodium deoxycholate to recover only low molecular weight LPS-containing fractions, whereby only a highly purified low molecular weight LPS is obtained by removal of the high molecular weight LPS from the fractions. The procedure of gel filtration in the presence of a surface-active agent is carried out to more highly purify the LPSs having a molecular weight of about 5,000 to about 6,000 which are disclosed in JP-A-4-187640, JP-A-4-49240 and JP-A-5-155778, whereby the high molecular weight LPS is completely removed from the fractions.

The term "crustaceans" used herein refers to all of lobsters, shrimps or prawns such as kuruma prawn (Penaeus japonicus), ushi prawn (Penaeus monodon), Yellow Sea prawn (Penaeus chinensis) and banana prawn (Penaeus morguiensis), and all of crabs such as Portunus trituberculatus and Chinese mitten crab, preferably lobsters, shrimps or prawns, more preferably prawns. The term "fishes" used herein include all of fishes such as yellowtail, globefish, real sea bream, flatfish, eel and rainbow trout. The infectious diseases referred to herein include acute viremia of crustaceans, their vivrio diseases, parasitosis such as Bpistylis sp., Zoothamnium sp. or mycosis such as Lagenidium sp., Siropidium sp.; iridovirus infectious diseases of fishes, their rhabdovirus diseases, neuronecrosis, infectious hemopoietic organ necrosis, pseudotuberculosis, streptococcic diseases, enterococcus diseases, vivrio diseases, cold-water disease, Pseudomonas diseases, gliding-bacteria diseases and Saprolegnia diseases, and all of infectious diseases caused by viruses, mycoplasmas, bacteria, fungi and parasites among which the feedstuff additive and feed of the invention can be more effectively used for viremia of crustaceans, and fishes' diseases such as streptococcic diseases, enterococcus diseases and vivrio diseases.

The low molecular weight LPS of the present invention can be used as a feed additive for crustaceans and fishes, and for this purpose, may be used as it is or as mixed with conventional carriers, stabilizers and the like and optionally with vitamins, amino acids, minerals and like nutrients, antioxidants, antibiotics, antibacterial agents and other additives. The feed additive is prepared in a suitable form such as powders, granules, pellets or suspensions. The feed additive may be supplied to crustaceans or fishes, alone or in mixture with a feed. For prevention of diseases, the feed additive may be supplied together with the feed at all times or at a latter half of feeding time.

The feeds of the present invention are not specifically limited but can be any of powdery feeds, solid feeds, moist pellet feeds, dry pellet feeds, extruder pellet feeds and live baits.

The proportion of the low molecular weight LPS in the feed of the invention can be selected from a wide range and is preferably 0.000001 to 0.001% by weight, more preferably 0.00002 to 0.00005% by weight to which its proportion is not limited. The amount of the low molecular weight LPS to be used can be suitably determined. For example, the LPS is applied at a daily dose of 1 to 100 µg, preferably 10 to 20 µg, per kilogram of the body weight of crustaceans or fishes to which, however, the dose is not limited.

### BEST MODE OF CARRYING OUT THE INVENTION

The present invention will be described in detail with reference to the following Examples to which, however, the invention is not limited. Low molecular weight LPS used in Examples is LPS having a molecular weight of about 5,000, and high molecular weight LPS is LPS having a molecular weight of about 8,000 to 50,000.

### Reference Example 1 (Preparation of low molecular weight LPS)

A 10 g quantity of tryptone (product of DIFCO CO.), 5 g of yeast extract (product of DIFCO CO.) and 10 g of NaCl (product of WAKO PURE CHEMICAL INDUSTRIES, LTD., special grade) were added to 1 liter of distilled water. The suspension was adjusted to a pH of 7.5 with NaOH and was sterilized in an autoclave. A single colony was separated from Pantoea agglomerance-carrying bacteria maintained at -80°C and was inoculated in a 500 ml-vol. Sakaguchi flask holding 100 ml of a culture medium containing sterile glucose (product of WAKO PURE CHEMICAL INDUSTRIES, LTD., special grade) at a proportion of 0.1% (hereinafter referred to as L-broth medium). Then the cells were subjected to shake culture at 35°C overnight. The cultured cells were inoculated in its entirety in a 3 liter-vol. Sakaguchi flask holding 1,000 ml of L-broth medium and were further cultivated in the same manner as above.

The cultured cells were inoculated in a 10-liter vol. desk fermenter (product of MARUBISHI BIOENGI CO.) holding 7 liters of L-broth medium and were subjected to aeration culture under the same conditions. The cells were collected to recover about 70 g of wet bacteria and were freeze-stored. About 70 g of freeze-stored cells were suspended in 500 ml of distilled water. A 500-ml quantity of 90% hot phenol was added to the suspension. The mixture was stirred at 65 to 70°C for 20 minutes and was cooled. The mixture was centrifuged at 10,000 G and 4°C for 20 minutes to recover the aqueous layer. The phenol layer was treated in the same manner as above. Then the two aqueous layers thus obtained were combined and dialyzed overnight to remove the phenol. The inner solution was concentrated by ultrafiltration in a 2 atom. nitrogen gas using an ultrafiltration device (product of ADVANTEC TOYO CO., K-200) with a membrane filter by cutting off molecular weight 200,000.

The lyophilized product of crude LPS thus obtained was dissolved in distilled water, the filter was sterilized, a buffer was added, and the solution was subjected to anion exchange chromatography (product of PHARMACIA Co., Q-Sepharose first flow). The specimen solution was passed through the column using a buffer containing 10 mM Tris-HCl (pH 7.5) and 10 mM NaCl to elute a limulus active fraction with 200 to 400 mM NaCl/10 mM Tris-HCL (pH 7.5). The eluate was subjected to ultrafiltration under the same conditions as above for desalting and concentration and was lyophilized to obtain about 300 mg of purified LPS from about 70 g of wet bacteria.

The obtained purified LPS (100 mg) was dissolved in a solubilizing buffer [comprising 3% sodium deoxycholate (product of WAKO PURE CHEMICAL INDUSTRIES LTD.), 0.2 M sodium chloride, 5 mM EDTA-2Na and 20 mM Tris-hydrochloric acid, pH 8.3]. The purified LPS solution (20 ml) was gently placed over Sephacryl S-200 HR column (product of PHARMACIA CO.). Then, 800 ml (50 hours) of the solution was eluted with an eluting buffer [comprising 0.25% sodium deoxycholate (product of WAKO PURE CHEMICAL INDUSTRIES LTD.), 0.2 M sodium chloride, 5 mM EDTA and 10 mM Tris hydrochoric acid, pH 8.3] at a flow velocity of 16 ml/hr.

The obtained eluate was fractionated by a fraction collector (product of ADVANTEC CO., trade name SF 2120) under control of flow velocity using a perista-pump PI (product of PHARMACIA CO.). A first 240-ml portion (24- fraction portion) was cast away. Thereafter the residue was fractionated into 80 fractions at 10 ml/fraction. The saccharide in the eluted fractions was quantitatively determined using the base solution or diluted solution by phenol/sulfuric acid method (Sakuzo FUKUI, "Method of Quantitative Determination of Reducing Sugar", 2nd ed., pp. 50 to 52, Gakkai Shuppan Center, 1990) to check the elution state. The fraction pattern of LPS was investigated by SDS-PAGE method using 0.5 ml of each of fractions 37 to 55 among the fractions presumably having LPS (fractions 30 to 60).

The result of investigation demonstrates that the fractions 45 to 55 contained only low molecular weight LPS (m.w. about 5000) and that fractions 37 to 44 contained both low molecular weight LPS and high molecular weight LPS. The low molecular weight LPS fractions of fractions 45 to 55 were further purified as follows.

The fractions was mixed, lyophilized and suspended in ethanol. The suspension was centrifuged to remove the deoxycholic acid soluble in ethanol and to recover a low molecular weight LPS in insoluble fractions. The ethanol treatment of the low molecular weight LPS fractions was further repeated twice, followed by removal of deoxycholic acid. The obtained LPS was suspended in 70% ethanol again, and the buffer component was removed by centrifugation. The same procedure was repeated three times for recovery of low molecular weight LPS in the insoluble fractions, followed by lyophilization, whereby about 20 mg of purified low molecular weight LPS was produced.

### Example 1 (Safety of low molecular weight LPS in crustaceans)

Kuruma prawns having an average weight of 20 g were divided into 5 groups of each 20 prawns. The low molecular weight LPS of the present invention was intramascularly administered to the third abdominal segment of prawns in Groups 1 and 2 at a dose of 50 mg and 100 mg, respectively per kilogram of the prawn's weight. On the other hand, a conventional high molecular weight LPS (derived from E. coli, E. coli 0111 manufactured by DIFCO CO.) was intramascularly administered to the third abdominal segment of prawns in Groups 3 and 4 at a dose of 10 mg and 20 mg, respectively per kilogram of the prawn's weight. Group 5 received a physiological saline free of LPS. The life or death of prawns up to 120 hours after administration was checked to determine a mortality. The results are shown in Table 1.

**Table 1**

| Group | number of perish / number tested | mortality (%) |
|---|---|---|
| Group 1 low MW LPS 50 mg/kg | 0/20 | 0 |
| Group 2 low MW LPS 100 mg/kg | 0/20 | 0 |
| Group 3 high MW LPS 10 mg/kg | 13/20 | 65 |
| Group 4 high MW LPS 20 mg/kg | 20/20 | 100 |
| Group 5 physiological saline | 0/20 | 0 |

As apparent from Table 1, a mortality of prawns in the groups receiving 10 mg or 20 mg of high molecular weight LPS was 65 or 100%, respectively, whereas no prawn died in the groups receiving 50 mg and 100 mg of low molecular weight LPS. It is clear from the above data that low molecular weight LPSs are significantly safe for prawns as compared with conventional high molecular weight LPSs.

### Example 2 (Safety of low molecular weight LPS in fishes)

Black carps having an average weight of 85 g were divided into 3 groups of each 40 carps. The low molecular weight LPS of the present invention was intramascularly administered to the dorsal region of black carps in Group 1 at a dose of 100 mg per kilogram of the carp's weight. On the other hand, a conventional high molecular weight LPS (trade name E. coli 0111 manufactured by DIFCO CO.) was intramascularly administered to the dorsal region of black carps in Group 2 at a dose of 20 mg per kilogram of the carp's weight. Group 3 received a physiological saline free of LPS. The life or death of black carps up to 120 hours after administration was checked to determine a mortality. The results are shown in Table 2.

**Table 2**

| Group | number of perish / number tested | mortality (%) |
|---|---|---|
| Group 1 low MW LPS 100 mg/kg | 0/40 | 0 |
| Group 2 high MW LPS 20 mg/kg | 34/40 | 85 |
| Group 3 physiological saline | 0/40 | 0 |

As apparent from Table 2, a mortality of black carps was 85% in the group receiving 20 mg of high molecular weight LPS, whereas no black carp died in the group receiving 100 mg of low molecular weight LPS. It is clear from the above data that the low molecular weight LPS of the present invention is significantly safe for fishes as compared with conventional high molecular weight LPS.

### Example 3 (Activity of activating phagocytosis in hemocyte of crustaceans)

Kuruma prawns having an average weight of 20 g were divided into 6 groups of each 20 prawns. Groups 1, 2 and 3 received the low molecular weight LPSs of the present invention as admixed with feeds at a daily dose of 20, 40 and 100 µg, respectively per kilogram of prawn's weight. On the other hand, Group 4 received a high molecular weight LPS as admixed with a feed at a daily dose of 100 µg, and Group 5 received the same at a daily dose of 1000 µg, per kilogram of prawn's weight.
The feeds were given for 7 days. Group 6 was given a feed free of LPS. On day 0, day 1, day 5 and day 7 after supply of the feeds, the blood was collected from the thorax recess of prawns using a syringe holding a K-199 culture medium containing L-cystein as an anticoagulant. Hemocyte cells were obtained by centrifugation. The obtained cells (1 × 10⁵ cells per microliter of the suspension) were mixed with 1 X 10⁸ latex beads (1.986 µm in diameter) and were reacted at 25°C for 30 minutes. After fixing the reaction mixture with glutaraldehyde, it was air-dried. Then the mixture was subjected to giemsa staining and was fixed to a slide glass with Eukitt. The same procedure was repeated to obtain five samples per prawn. The hemocyte cells (200 cells per sample) were observed at random under an epi-fluorescent microscope to determine the phagocytosis ratio of latex beads in hemocyte and the number of latex beads phagocytized into one cell of hemocyte. Then the phagocytosis index was calculated by the following equation. Phagocytosis ratio=[number of hemocyte cells taking beads/total number of hemocyte cells observed] × 100.
Average number of beads taken by hemocyte cells = number of beads taken by hemocyte cells/number of hemocyte cells taking beads.
Phagocytosis index= [number of hemocyte cells taking beads/total number of hemocyte cells observed] × [number of beads taken by hemocyte cells/total number of hemocyte cells observed] × 100. Test results: The biophylaxis of crustaceans involves a cell factor and a liquid factor. The phagocytosis of foreign particles in hemocyte is deeply concerned with the former.
When the phagocytosis of foreign particles in prawn's hemocyte is assessed, it is clarified whether the defensive mechanism of prawns is activated. [Yukinori TAKAHASHI et al, Research of Fish Diseases, 30 (2), pp.141 to 150, (1995)]. In view of said theory, the phagocytosis index was determined on day 0, day 1, day 5 and day 7 after supply of feeds for the groups receiving high molecular weight LPSs and the groups receiving the low molecular weight LPSs. The results were tabulated in Table 3.

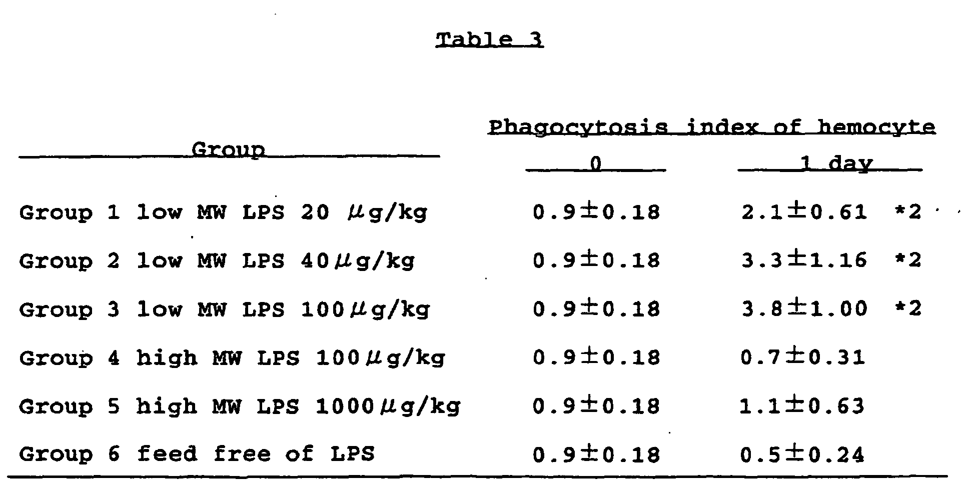

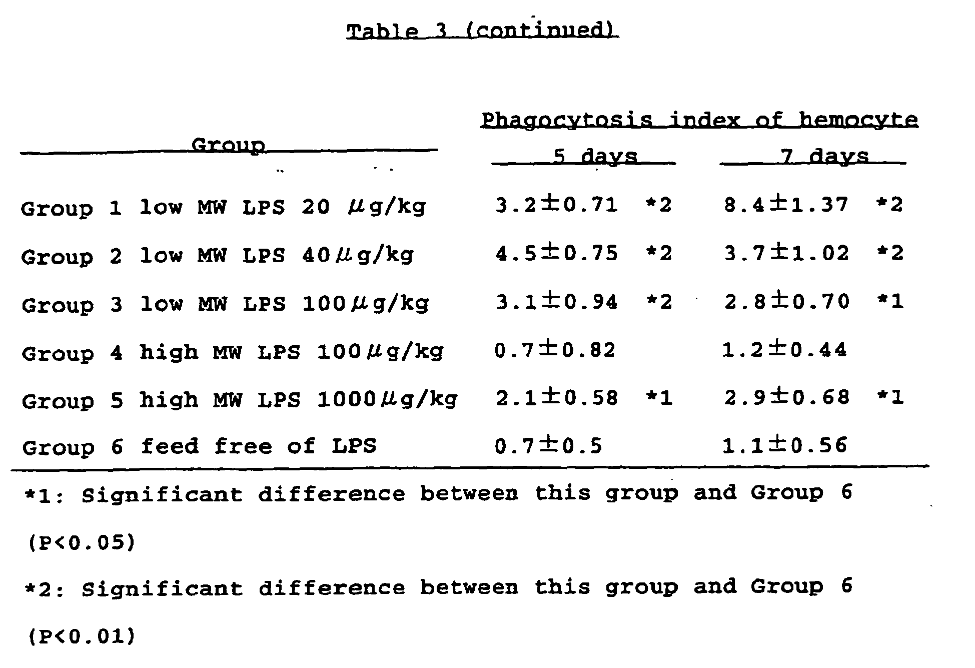

As apparent from Table 3, the groups receiving the low molecular weight LPSs (present invention) showed a higher phagocytosis index in hemocyte of prawns than Group 6 and a significant difference in this index from Group 6 (P<0.01, P<0.05). The group receiving 100 µg of conventional high molecular weight LPS was unable to increase the phagocytosis index in hemocyte of prawns after 1, 5 and 7 days. However, the group receiving 1000 µg of conventional high molecular weight LPS showed a significantly higher phagocytosis index in hemocyte of prawns (P<0.05) than Group 6 after 5 and 7 days.
The above data show that the low molecular weight LPSs of the present invention can activate the defensive mechanism such as phagocytosis in hemocyte of prawns even when used in an extremely smaller amount than the high molecular weight LPSs. Example 4 (Activity of activating phenol oxidase in hemocyte of crustaceans)

Kuruma prawns having an average weight of 20 g were divided into 6 groups of each 20 prawns. Groups 1, 2 and 3 received the low molecular weight LPSs of the present invention as admixed with feeds at a daily dose of 20, 40 and 100 µg, respectively per kilogram of prawn's weight. Group 4 received a high molecular weight LPS as admixed with a feed at a daily dose of 100 µg, and Group 5 received the same as admixed with a feed at a daily dose of 1000 µg, per kilogram of prawn's weight. The supply of the feeds continued for 7 days. Group 6 received a LPS-free feed. The blood was collected from the thorax recess of prawns using a syringe holding a KHE culture medium having EDTA on day 0, day 1, day 5 and day 7 after supply of feeds. The collected blood was centrifuged to obtain hemocyte cells. The obtained cells were suspended in a Ca-Mg Hepes culture medium to a concentration of 1 x 10⁶ cells/ml. The cells were crushed by freeze resolution and supersonic waves. The supernatant was separated off by centrifugation and was filtered with a membrane filter. The obtained filtrate (900 µl) was mixed with 100 µl of L-DOPA solution as a substrate solution. Thereafter the mixture was reacted at a temperature of 60°C for 60 minutes. Then the absorbance at 490 nm was measured by a spectrophotometer to assess a phenol oxidase activity (PO activity).

Test results: The biophylaxis of crustaceans involves a cell factor and a liquid factor. The PO activity in hemocyte is deeply concerned with the latter. Thus, it is clarified by assessment of PO activity whether the defensive mechanism of prawns is activated. The PO activity of prawns was determined on day 0, day 1, day 5 and day 7 after supply of feeds for the groups receiving the low molecular weight LPSs (present invention) and the groups receiving high molecular weight LPSs. The results were tabulated in Table 4.

**Table 4**

| Group | PO activity (absorbance·490nm) | | | | | | |
|---|---|---|---|---|---|---|---|
| | 0 | 1 day | | 5 days | | 7 days | |
| Group 1 low MW | 0.092 | 0.105 | | 0.199 | *1 | 0.405 | *2 |
| LPS 20 µg/kg | | | | | | | |
| Group 2 low MW | 0.092 | 0.115 | | 0.201 | *1 | 0.325 | *2 |
| LPS 40µg/kg | | | | | | | |
| Group 3 low MW | 0.092 | 0.166 | *1 | 0.170 | *1 | 0.292 | *2 |
| LPS 100µg/kg | | | | | | | |
| Group 4 high MW | 0.092 | 0.093 | | 0.124 | | 0.138 | |
| LPS 100µg/kg | | | | | | | |
| Group 5 high MW | 0.092 | 0.104 | | 0.197 | *1 | 0.230 | *1 |
| LPS 1000µg/kg | | | | | | | |
| Group 6 feed free of LPS | 0.092 | 0.093 | | 0.136 | | 0.123 | |

| | | | | | | | |
|---|---|---|---|---|---|---|---|
| *1: significant difference between this group and Group 6 (P<0.05) *2: significant difference between this group and Group 6 (P<0.01) | | | | | | | |

As apparent from Table 4, the groups receiving the low molecular weight LPSs (present invention) indicated a higher PO activity than Group 6 and a significant difference in this activity from Group 6 (P<0.01, P<0.05). The group receiving 100 µg of conventional high molecular weight LPS did not increase in PO activity in hemocyte of prawns up to 7 days.
The group receiving 1000 µg of conventional high molecular weight LPS showed a significantly higher PO activity in hemocyte of prawns(P<0.05) than Group 6 after 5 and 7 days.
The above data show that the low molecular weight LPSs of the present invention can activate the defensive mechanism such as PO activity in hemocyte of prawns even when used in an extremely smaller amount than the high molecular weight LPSs.

### Example 5 (Effect of preventing acute viremia in kuruma prawns)

Kuruma prawns having an average weight of 14 g were divided into 7 groups of each 20 prawns. Groups 1, 2 and 3 received the low molecular weight LPSs of the present invention as admixed with feeds at a daily dose of 20, 40 and 100 µg, respectively per kilogram of prawn's weight. Group 4 received a high molecular weight LPS as admixed with a feed at a daily dose of 1000 µg, per kilogram of prawn's weight. Group 5 received peptidoglycan (PG) derived from Bifidobacterium thermophilum (Patent No.2547371) as admixed with a feed at a daily dose of 0.2mg (200 µg), per kilogram of prawn's weight. Group 6 received β-1,3-glucan (1,3-G) derived from Schizophyllum commune (JP-B-6-65649) as admixed with a feed at a daily dose of 50mg (50000 µg), per kilogram of prawn's weight. The supply of feeds continued for 18 days. Group 7 (control group) was given a LPS-free feed.

On day 8 after the start of supply of LPS, infection test was conducted using PRDV (penaeid rod-shaped DNA virus) as a pathogen inducing acute viremia in prawns. Carapaces were removed from the cephalothorax of three prawns which died of acute viremia. The intestine of prawns was crushed and homogenized in 40 ml of sterile seawater. The supernatant (10 ml) was separated off by centrifugation (10,000 × g, 10 minutes, 4°C) and added to 20 liters of seawater. On day 8 after the start of supply of LPS, prawns were infected with acute viremia by immersion in the supernatant for 2 hours. The life or death of prawns was observed for 10 days after infection. The dead prawns were pathologically tested and examined by PCR (polymerase chain reaction) method to confirm whether the prawns died of infection with PRDV.

Test results: Tables 5 and 6 show the total number of dead prawns and a mortality after infection with PRDV in the groups receiving low molecular weight LPSs of the present invention, the group receiving a high molecular weight LPS and the group receiving a LPS-free feed.

**Table 5**

| Group | Days after infection | | | | |
|---|---|---|---|---|---|
| | 1 | 2 | 3 | 4 | 5 |
| Group 1 low MW | 0 | 0 | 0 | 2* | 3 |
| LPS 20 µg/kg | | | | | |
| Group 2 low MW | 0 | 0 | 3 | 4 | 4 |
| LPS 40µg/kg | | | | | |
| Group 3 low MW | 1 | 1 | 3 | 3 | 4 |
| LPS 100µg/kg | | | | | |
| Group 4 high MW | 1 | 1 | 6 | 6 | 6 |
| LPS 1000µg/kg | | | | | |
| Group 5 | 0 | 0 | 2 | 5 | 5 |
| PG 0.2mg/kg | | | | | |
| Group 6 | 0 | 3 | 5 | 7 | 10 |
| 1,3-G 50mg/kg | | | | | |
| Group 7 feed free of LPS | 2 | 4 | 13 | 14 | 15 |

| | | | | | |
|---|---|---|---|---|---|
| * The number indicates the total number of dead prawns. (Other numbers show the same.) | | | | | |

**Table 6**

| Group | Days after infection | | | | | Mortality |
|---|---|---|---|---|---|---|
| | 6 | 7 | 8 | 9 | 10 | |
| Group 1 low MW | 3 | 3 | 4 | 4 | 4 | 20 *** |
| LPS 20 µg/kg | | | | | | |
| Group 2 low MW | 6 | 6 | 6 | 7 | 7 | 35 *** |
| LPS 40µg/kg | | | | | | |
| Group 3 low MW | 5 | 6 | 8 | 8 | 8 | 40 *** |
| LPS 100µg/kg | | | | | | |
| Group 4 high MW | 9 | 9 | 10 | 11 | 11 | 55 ** |
| LPS 1000µg/kg | | | | | | |
| Group 5 | 7 | 8 | 8 | 8 | 10 | 50 ** |
| PG 0.2mg/kg | | | | | | |
| Group 6 | 10 | 11 | 11 | 12 | 12 | 60 ** |
| 1,3-G 50mg/kg | | | | | | |
| Group 7 feed free of LPS | 18 | 18 | 19 | 20 | 20 | 100 |

| | | | | | | |
|---|---|---|---|---|---|---|
| ** : significant difference between this group and Group 7 (P<0.05) *** : significant difference between this group and Group 7 (P<0.01) | | | | | | |

All (100%) of prawns died in the control group receiving a LPS-free feed up to 9 days after infection with PRDV. On the other hand, 20%, 35% and 40% of prawns died in the groups receiving 20, 40 and 100 µg, respectively of low molecular weight LPS (present invention). In other words, a low mortality resulted from these groups, and a significant difference (P<0.01) exists between these groups and the control group. In contrast, 55% of prawns died in the group receiving 1000 µg of high molecular weight LPS, which means that more prawns died in this group than the groups receiving the low molecular weight LPSs. The above data demonstrate that the low molecular weight LPSs of the present invention can prevent viral infection of prawns and that the low molecular weight LPSs are more efficacious than conventional high molecular weight LPSs.

### Example 6 (Activity of activating immune function in fishes)

Yellowtails weighing 230 g on an average were divided into 6 groups of each 20 yellowtails. Groups 1, 2 and 3 received the low molecular weight LPSs of the present invention as admixed with moist pellets at a daily dose of 20, 40 and 100 µg, respectively per kilogram of yellowtail's weight. Group 4 received a high molecular weight LPS as admixed with moist pellets at a daily dose of 100 µg, and Group 5 received a high molecular weight LPS as admixed with moist pellets at a daily dose of 1000 µg, per kilogram of yellowtail's weight. The feeds were given for 7 days. Group 6 received LPS-free moist pellets. On day 0, day 1, day 5 and day 7 after supply of feeds, a head kidney was excised from 5 yellowtails. Then hemocyte cells were separated in a plastic petri dish holding a 0.25% NaCl-containng RPMI-1640-HAH culture medium. The cells were passed through a cell filter to give a cell suspension. The suspension was placed over a discontinuous Percoll density gradient. Thereafter a leukocyte layer was formed by centrifugation (1600 rpm., at 4°C for 20 minutes).

The leukocyte layer was collected and was subjected to centrifugal washing after which the cells were suspended in a 10% FBS (fetal bovine serum)-containing 0.25% NaCl-including RPMI-1640-H culture medium. The number of leukocyte cells in the suspension was adjusted to 1 X 10⁶ cells/ml. The leukocyte suspension (500 µl) and 500 µl of a suspension (1 × 10⁸ cells/ml) of yeast opsonized with serum of yellowtail were placed into a silicone-treated glass test tube and were incubated at 25°C for 60 minutes with stirring every 10 minutes. After incubation, 5 smears per yellowtail were produced, subjected to Wright's staining and enclosed with Eukitt. The hemocyte cells (200 cells per smear) were observed at random under an optical microscope. Then the number of yeast cells phagocytized into leukocyte was counted. The phagocytosis index was given by the same equation as in Example 3. The results are shown in Tables 7 and 8.

**Table 7**

| Group | Phagocytosis index of leukocyte | | |
|---|---|---|---|
| | 0 | 1 day | |
| Group 1 low MW LPS 20 µg/kg | 7.3±2.30 | 12.7±2.65 | *1 |
| Group 2 low MW LPS 40µg/kg | 7.3±2.30 | 17.9±3.99 | *2 |
| Group 3 low MW LPS 100µg/kg | 7.3±2.30 | 18.6±4.12 | *2 |
| Group 4 high MW LPS 100µg/kg | 7.3±2.30 | 6.3±2.24 | |
| Group 5 high MW LPS 1000µg/kg | 7.3±2.30 | 8.2±2.18 | |
| Group 6 feed free of LPS | 7.3±2.30 | 6.6±1.19 | |

| | | | |
|---|---|---|---|
| *1: significant difference between this group and Group 6 (P<0.05) *2: significant difference between this group and Group 6 (P<0.01) | | | |

**Table 8**

| Group | Phagocytosis index of leukocyte | | | |
|---|---|---|---|---|
| | 5 days | | 7 days | |
| Group 1 low MW LPS 20 µg/kg | 39.2±2.54 | *2 | 52.7±4.08 | *2 |
| Group 2 low MW LPS 40µg/kg | 37.4±4.28 | *2 | 37.0±3.11 | *2 |
| Group 3 low MW LPS 100µg/kg | 42.6±5.35 | *2 | 36.5±4.32 | *1 |
| Group 4 high MW LPS 100µg/kg | 11.2±3.05 | | 10.6±2.96 | |
| Group 5 high MW LPS 1000µg/kg | 22.7±3.16 | *1 | 31.8±3.52 | *1 |
| Group 6 feed free of LPS | 9.0±2.04 | | 7.7±1.73 | |

| | | | | |
|---|---|---|---|---|
| *1: significant difference between this group and Group 6 (P<0.05) **2: significant difference between this group and Group 6 (P<0.01) | | | | |

As apparent from Tables 7 and 8, any groups of yellowtails receiving the low molecular weight LPSs (present invention) indicated a higher phagocytosis index in leukocyte of yellowtails than Group 6 and a significant difference (P<0.01, P<0.05) in this index from Group 6. However, the group receiving 100 µg of conventional high molecular weight LPS did not increase the phagocytosis index in leukocyte of yellowtails after 7 days. The group receiving 1000 µg of conventional high molecular weight LPS showed a significantly higher phagocytosis index (P<0.01) in leukocyte of yellowtails than Group 6 after 5 days. The above data show that the low molecular weight LPSs of the present invention can activate the immune system of fishes such as phagocytosis in leukocyte in an extremely smaller amount than conventional high molecular weight LPSs.

### Example 7 (Effect of preventing enterococcus disease in yellowtails)

Yellowtails weighing 63 g on an average were divided into 5 groups of each 30 yellowtails. Groups 1, 2 and 3 received the low molecular weight LPSs of the present invention as admixed with moist pellets at a daily dose of 20, 40 and 100 µ g, respectively per kilogram of yellowtail's weight. Group 4 received a high molecular weight LPS as admixed with moist pellets at a daily dose of 1000 µg per kilogram of yellowtail's weight. Group 5 (control) received LPS-free moist pellets. On day 7 after supply of feeds, the yellowtails were intraabdominally inoculated with Enterococcus Seriolicida as a pathogen causing enterococcus disease of yellowtail in an amount of 4.0 X 10⁶ cells per yellowtail. A mortality 15 days after inoculation was determined. The results are shown in Tables 9 and 10.

**Table 9**

| Group | Days after infection | | | | | | | | |
|---|---|---|---|---|---|---|---|---|---|
| | 1 | 2 | 3 | 4 | 5 | 6 | 7 | 8 | 9 |
| Group 1 low MW | 0 | 0 | 0 | 0 | 0 | 0 | 0 | 0 | 1* |
| LPS 20 µg/kg | | | | | | | | | |
| Group 2 low MW | 0 | 0 | 0 | 1 | 1 | 2 | 2 | 4 | 4 |
| LPS 40µg/kg | | | | | | | | | |
| Group 3 low MW | 0 | 0 | 0 | 0 | 0 | 1 | 3 | 3 | 5 |
| LPS 100µg/kg | | | | | | | | | |
| Group 4 high MW | 0 | 0 | 0 | 1 | 1 | 1 | 3 | 3 | 3 |
| LPS 1000µg/kg | | | | | | | | | |
| Group 5 feed | 0 | 0 | 1 | 2 | 7 | 7 | 10 | 12 | 16 |
| free of LPS | | | | | | | | | |

| | | | | | | | | | |
|---|---|---|---|---|---|---|---|---|---|
| * The number indicates the total number of dead yellowtails. (Other numbers show the same.) | | | | | | | | | |

**Table 10**

| Group | Days after infection | | | | | | Mortality | |
|---|---|---|---|---|---|---|---|---|
| | 10 | 11 | 12 | 13 | 14 | 15 | (%) | |
| Group 1 low MW | 3 | 3 | 3 | 3 | 4 | 4 | 13.3 | *** |
| LPS 20 µg/kg | | | | | | | | |
| Group 2 low MW | 7 | 8 | 8 | 8 | 8 | 8 | 26.7 | ** |
| LPS 40µg/kg | | | | | | | | |
| Group 3 low MW | 5 | 5 | 5 | 7 | 7 | 7 | 23.3 | ** |
| LPS 100µg/kg | | | | | | | | |
| Group 4 high MW | 5 | 9 | 10 | 10 | 11 | 11 | 36.7 | ** |
| LPS 1000µg/kg | | | | | | | | |
| Group 5 feed | 16 | 16 | 17 | 22 | 22 | 22 | 73.3 | |
| free of LPS | | | | | | | | |

| | | | | | | | | |
|---|---|---|---|---|---|---|---|---|
| ** : significant difference between this group and Group 5 (P<0.05) ***: significant difference between this group and Group 5 (P<0.01) | | | | | | | | |

On 15th day after inoculation of E. Seriolicida, 73.3% of yellowtails died in the control group receiving LPS-free feed. In contrast, a low mortality is indicated by the groups receiving the low molecular weight LPSs of the present invention, i.e. 13.3% from the group receiving 20 µg, 26.7% from the group receiving 40 µg and 23.3% from the group receiving 100 µg. In other words, there is a significant difference (P<0.05) in mortality between these groups and the control group. On the other hand, a mortality of 36.7% resulted from the group receiving 1000 µg of high molecular weight LPS. This group showed a higher mortality than the groups receiving low molecular weight LPSs. The above results show that the low molecular weight LPSs of the present invention can protect fishes against viral infection and are more efficacious than conventional high molecular weight LPSs.

### INDUSTRIAL APPLICABILITY

According to the present invention, there is provided a safe feedstuff additive for growing crustaceans and fishes, the feedstuff additive being capable of preventing infectious diseases by properly activating their intrinsic immune function even when used in a small amount, being capable of preventing the perish of crustaceans and fishes, and being free from the problems of public health hazards such as the feedstuff additive remaining in crustaceans and fishes.

## Claims

1. A feedstuff additive for crustaceans or fishes, **characterized in that** it is prepared from gram-negative bacteria, that it is substantially free of high molecular weight lipopolysaccharide, that it contains, as an effective component, a low molecular weight lipopolysaccharide having a molecular weight of 5000 +/- 2000 as measured by SDS-PAGE method using a protein marker, and that it is capable of activating immunity or preventing infection in crustaceans or fishes.

2. A feedstuff additive for crustaceans or fishes comprising the low molecular weight lipopolysaccharide of claim 1 and a carrier acceptable for crustaceans and fishes.

3. Use of the low molecular weight lipopolysaccharide of claim 1 for the preparation of a feedstuff additive for crustaceans or fishes.

4. An agent for preventing the perish of crustaceans or fishes comprising the low molecular weight lipopolysaccharide of claim 1 as an effective component.

5. An agent for preventing the perish of crustaceans or fishes according to claim 4 further comprising a carrier acceptable for crustaceans and fishes.

6. Use of the low molecular weight lipopolysaccharide of claim 1 for the preparation of an agent for preventing the perish of crustaceans or fishes.

7. A feedstuff additive according to claim 1, wherein the gram-negative bacteria are those pertaining to genus Pantoea.

8. A feedstuff additive according to claim 7, wherein the gram-negative bacteria are Pantoea agglomerans.

9. A feed for crustaceans or fishes comprising the feedstuff additive of claim 1.

10. A feedstuff additive according to claim 1, wherein the infectious diseases is acute viremia of crustaceans, their vibrio diseases, parasitosis or mycosis; iridovirus infectious diseases of fishes, their rhabdovirus diseases, neuronecrosis, infectious hemopoietic organ necrosis, pseudotuberculosis, streptococcic diseases, enterococcus diseases, vibrio diseases, cold-water disease, Pseudomonas diseases, gliding-bacteria diseases or Saprolegnia diseases.

11. A feedstuff additive according to claim 1, wherein the high molecular weight lipopolysaccharide is one having a molecular weight of at least 8,000.

## Patentansprüche

1. Futtermittelzusatz für Krustentiere oder Fische, **dadurch gekennzeichnet, dass** er hergestellt ist aus Gram-negativen Bakterien, dass er im wesentlichen frei ist von hochmolekularem Lipopolysaccharid, dass er als wirksame Komponente ein niedermolekulares Lipopolysaccharid mit einem Molekulargewicht von 5000 +/- 2000, gemessen nach der SDS-PAGE-Methode unter Verwendung eines Proteinmarkers, enthält und dass er fähig ist, in Krustentieren oder Fischen die Immunität zu aktivieren und Infektionen zu verhindern.

2. Futtermittelzusatz für Krustentiere oder Fische, umfassend das niedermolekulare Lipopolysaccharid von Anspruch 1 und einen für Krustentiere und Fische annehmbaren Träger.

3. Verwendung des niedermolekularen Lipopolysaccharids von Anspruch 1 zur Herstellung eines Futtermittelzusatzes für Krustentiere und Fische.

4. Mittel zur Verhinderung des Verderbs von Krustentieren und Fischen, umfassend das niedermolekulare Lipopolysaccharid von Anspruch 1 als wirksame Komponente.

5. Mittel zur Verhinderung des Verderbs von Krustentieren und Fischen nach Anspruch 4, ferner umfassend einen für Krustentiere und Fische annehmbaren Träger.

6. Verwendung des niedermolekularen Lipopolysaccharids von Anspruch 1 zur Herstellung eines Mittels zur Verhinderung des Verderbs von Krustentieren und Fischen.

7. Futtermittelzusatz nach Anspruch 1, worin die Gram-negativen Bakterien zum Genus Pantoea gehören.

8. Futtermittelzusatz nach Anspruch 7, worin die Gram-negativen Bakterien Pantoea agglomerans sind.

9. Futter für Krustentiere oder Fische, umfassend den Futtermittelzusatz von Anspruch 1.

10. Futtermittelzusatz nach Anspruch 1, worin die Infektionskrankheiten akute Krustentier-Virämie, deren Vibrio-Krankheiten, Parasitose oder Mykose, lridovirus-Infektionskrankheiten von Fischen, deren Rhabdovirus-Krankheiten, Neuronekrose, infektiöse Hämopoeseorgan-Nekrose, Pseudotuberkulose, Streptokokken-Krankheiten, Enterokokken-Krankheiten, Vibrio-Krankheiten, Kaltwasser-Krankheit, Pseudomonas-Krankheiten, Gleitbakterien-Krankheiten oder Saprolegnia-Krankheiten sind.

11. Futtermittelzusatz nach Anspruch 1, worin das hochmolekulare Lipopolysaccharid ein Molekulargewicht von mindestens 8000 hat.

## Revendications

1. Additif alimentaire pour crustacés et poissons, **caractérisé en ce qu'**il est préparé à partir de bactéries à Gram négatif, qu'il ne contient pratiquement pas de lipopolysaccharides de masse moléculaire élevée, qu'il contient, en tant que composant efficace, un lipopolysaccharide de faible masse moléculaire possédant une masse molaire, mesurée par SDS-PAGE à l'aide d'un marqueur protéique, de 5000 ± 2000, et qu'il est capable d'activer l'immunité ou de prévenir une infection chez des crustacés ou des poissons.

2. Additif alimentaire pour crustacés et poissons, comprenant un lipopolysaccharide de faible masse moléculaire, défini dans la revendication 1, et un véhicule admissible pour crustacés et poissons.

3. Emploi d'un lipopolysaccharide de faible masse moléculaire, défini dans la revendication 1, en vue de la préparation d'un additif alimentaire pour crustacés et poissons.

4. Agent de prévention de mort par maladie de crustacés ou de poissons, comprenant un lipopolysaccharide de faible masse moléculaire, défini dans la revendication 1, en tant que composant efficace.

5. Agent de prévention de mort par maladie de crustacés ou de poissons, conforme à la revendication 4, comprenant en outre un véhicule admissible pour crustacés et poissons.

6. Emploi d'un lipopolysaccharide de faible masse moléculaire, défini dans la revendication 1, en vue de la préparation d'un agent de prévention de mort par maladie de crustacés ou de poissons.

7. Additif alimentaire conforme à la revendication 1, pour lequel les bactéries à Gram négatif appartiennent au genre *Pantoea*.

8. Additif alimentaire conforme à la revendication 7, pour lequel les bactéries à Gram négatif sont des bactéries *Pantoea agglomerans*.

9. Aliment pour crustacés ou poissons, comprenant un additif alimentaire conforme à la revendication 1.

10. Additif alimentaire conforme à la revendication 1, la maladie infectieuse étant l'une des suivantes : chez les crustacés, virémie aiguë, maladies à *Vibrio*, parasitoses et mycoses, et chez les poissons, maladies infectieuses à iridovirus, maladies à rhabdovirus, neuronécrose, nécrose infectieuse des organes hématopoïétiques, pseudotuberculose, maladies à streptocoques, maladies à entérocoques, maladies à *Vibrio*, maladies des eaux froides, maladies à *Pseudomonas*, maladies à bactéries glissantes et maladies à *Saprolegnia*.

11. Additif alimentaire conforme à la revendication 1, lesdits lipopolysaccharides de masse moléculaire élevée étant ceux qui présentent une masse molaire d'au moins 8000.
